# EUROPÄISCHE PATENTSCHRIFT

(11) **EP 0 393 539 B1**
(45) Veröffentlichungstag und Bekanntmachung des Hinweises auf die Patenterteilung: **04.08.1993**
(21) Anmeldenummer: 90107125.8
(22) Anmeldetag: 13.04.1990
(51) Int. Cl.: A61K 31/565, A61K 9/48, A61K 9/66

(54) **Orale Zubereitungsform des Östradiolvalerats**
Oral preparation containing estradiol valerate
Préparation orale contenant le valérate d'estradiol

(30) Priorität: 18.04.1989 DE 3912625; 03.06.1989 DE 3918148
(43) Veröffentlichungstag der Anmeldung: 24.10.1990
(73) Patentinhaber: HENNING BERLIN GmbH CHEMIE- UND PHARMAWERK, D-12099 Berlin (DE); R.P. Scherer GmbH, D-69412 Eberbach (DE)
(72) Erfinder: Fischer, Gerhard, Dr., D-6930 Eberbach (DE); Decker, Günter, Dr., D-1000 Berlin 12 (DE)
(74) Vertreter: Werner, Hans-Karsten, Dr.Dipl.-Chem.

(56) Entgegenhaltungen:
- DE-A- 1 792 778
- DE-A- 2 758 549
- FR-A- 2 269 925
- FR-A- 2 312 256

## Beschreibung

Gegenstand der vorliegenden Erfindung ist eine orale Zubereitungsform des Östradiolvalerats zur postmenopausalen Substitutionstherapie sowie der Prophylaxe und Therapie der Osteoporose. Zur postmenopausalen Substitutionstherapie sowie der Prophylaxe und Therapie der Osteoporose werden in zunehmendem Maße Östrogene empfohlen, wobei diese im Wechselrhythmus mit Gestagenen genommen werden sollen, um möglichst weitgehend den physiologischen Rhythmus aus der Zeit vor der Menopause aufrechtzuerhalten. Als Östrogene werden zur Empfängnisverhütung vor allem synthetische Östrogene wie zum Beispiel Mestranol oder konjugierte Östrogene wie Presomen verwendet. Weiterhin kommt aber auch Östradiol in mikronisierter Form oder als Östradiolvalerat zur Anwendung. Zur postmenopausalen Substitutionstherapie und Prophylaxe der Osteoporose wird vorzugsweise Östradiol mikronisiert oder Östradiolvalerat empfohlen, da es sich um das natürliche Östrogen der Frau handelt und deshalb hiermit tatsächlich nur der postmenopausale Östrogenmangel kompensiert wird.

Aus der DE-OS 27 58 549 ist bekannt, daß Östradiolvalerianat auch eine neuropsychotrope Wirkung, insbesondere mit antidepressiver Wirkung, aufweist. Dazu kann der Wirkstoff in an sich bekannter Weise galenisch zubereitet und appliziert werden. Die Beispiele beschreiben übliche Tabletten, ölige Injektionslösungen und Hartgelatinesteckkapseln.

Aus der DE-OS 30 07 251 sind oral applizierbare Arzneiformen von Alkylsulfonsäureestern des Ethinylöstradiols bekannt, welche den Wirkstoff in einem lipophilen Lösungs mittel und einem Co-Solvens, wie Benzylalkohol, enthalten. Dieses Lösungsmittelsystem soll eine höhere Bioverfügbarkeit haben, die nahezu die Bioverfügbarkeit intravenöser Application erreicht. Es handelt sich somit um einen Wirkstoff mit sehr hoher Resorbierbarkeit.

Aus der umfangreichen Literatur auf dem Gebiet der Östrogene geht hervor, daß etwa 50 µg synthetische Östrogene wie Mestranol, 1,25 mg konjugierte Östrogene wie zum Beispiel Presomen sowie 2 mg Östradiol mikronisiert oder als Valerat dosisäquivalent sind. Um mögliche Nebenwirkungen durch Überdosierung zu vermeiden, werden für Östradiolvalerat Dosen von 1 bis 3 mg empfohlen. Aus einer Placebo-kontrollierten Studie von Christiansen et al. geht hervor, daß 1 mg Östradiol (mikronisiert) ausreichend ist, den Knochenmineralverlust zu vermeiden. Die Östradiol-Serumspiegel nach 12-monatiger Behandlung lagen dabei im Mittel um 75 pg/ml (entsprechend 250 p mol/L). Bei Verwendung von 2 mg mikronisiertem Östradiol wurde nicht nur der Knochenverlust gestoppt, sondern nahm die Knochenmasse sogar um 1% pro Jahr zu. Bei Verwendung von 4 mg Östradiol betrug die Zunahme der Knochenmasse ca. 2%. Die Östradiol-Serumspiegel lagen nach 12 Monaten Therapie bei ca. 100 bzw. 140 pg/ml (vgl. Christensen et al., Am. J. Obstet. Gynecol. 144 (1982) 873-879 und Christiansen et al., Journ. of Clin. Endocrinology and Metabolism 55 (1982) 1124-1130). Aus Studien über die Pharmakokinetik von Östradiolvalerat geht hervor, daß sich die Östradiol-Serumspiegel bei postmenopausalen Frauen während einer 21-tägigen Einnahme von täglich 2 mg Östradiolvalerat zwischen 50 und maximal 150 pg/ml bewegen (vgl. Englund et al., Acta Obstet. Gynecol. Scand. Suppl. 65 (1977) 27-31 und Düsterberg et al., Hormone Res. 21 (1985) 145-154). Hierbei ist zu beachten, daß 2 mg Östradiolvalerat etwa 1,5 Östradiol entsprechen.

Aus diesen Studien geht hervor, daß Östradiolvalerat rasch absorbiert wird und höhere Östradiolspiegel liefert als die entsprechende Menge Östradiol in der dort verwendeten Galenik. Aus den Untersuchungen von Düsterberg geht hervor, daß die Bioverfügbarkeit von oral genommenen Steroidestern relativ gering ist und nur etwa 3% des oral eingenommenen Östradiolvalerats als unverändertes Östradiol peripher zur Wirkung kommen. Es ist daher nötig, oral wesentlich höhere Mengen an Östradiol bzw. Östradiolvalerat zu geben als bei intramuskulärer Injektion (vgl. Düsterberg, a.a.O., Seite 153).

Die Erfindung hat sich die Aufgabe gestellt, eine orale Zübereitungsform des Östradiolvalerats zu entwickeln, die gut verträglich und zuverlässig zur postmenopausalen Substitutionstherapie sowie der Prophylaxe und Therapie der Osteoporose verwendet werden kann.

Es wurde jetzt gefunden, daß diese Aufgabe überraschend einfach gelöst werden kann durch Weichgelatinekapseln enthaltend 0,5 bis 1,5 mg Östradiovalerat gelöst in einem physiologisch verträglichen lipophilen Medium, insbesondere in einem Pflanzenöl, vorzugsweise Soja- und/oder Sesamöl.

Aus bisher unerklärlichen Gründen führt diese orale Zubereitungsform zu einer etwa 6%-igen Bioverfügbarkeit des Östradiols in der Peripherie, wobei überraschenderweise die Anflutung des Wirkstoffes wesentlich gleichmäßiger erfolgt als bei der bisher üblichen Applikation von Östradiolvalerat in Form von Tabletten. Diese höhere Bioverfügbarkeit, verbunden mit der gleichmäßigeren Anflutung des Wirkstoffes, gestattet es, die Dosis zu halbieren und dennoch gleichmäßig hohe periphere Serumspiegel zu erzielen. Die gleichmäßigere Anflutung des Wirkstoffes führt dabei zu einer besonders guten Verträglichkeit des Präparates, da anscheinend auch die tageszeitlichen Schwankungen des peripheren Serumspiegels zu gewissen Unverträglichkeiten beitragen.

Erfindungsgemäße Weichgelatinekapseln mit einem Gehalt von 1 mg Östradiolvalerat führen bereits zu durchschnittlichen Serumspiegeln von 150 bis 200 pg/ml. Bereits die Verwendung von Weichgelatinekapseln mit 1,5 mg Östradiolvalerat führt zu Serumspiegeln von 300 bis 400 pg/ml, wie sie physiologisch nur in der periovulatorischen Phase gefunden werden und nicht mehr mit einer physiologischen Östradiolsubstitution vereinbar sind. Im Vergleich hierzu wurde gefunden, daß bei oraler Gabe von 2 mg mikronisiertem Östradiol die Östradiolspiegel auf maximal 120 pg/ml ansteigen.

Untersuchungen der Pharmakokinetik haben ergeben, daß bei der erfindungsgemäßen oralen Zubereitungsform die peripheren Serumspiegel bereits nach ca. 1 Stunde erreicht werden und dann über den weiteren Tagesverlauf hin ziemlich gleichmäßig aufrechterhalten werden. Vergleichende Untersuchungen mit mikronisiertem Östradiol haben ergeben, daß mit diesen Präparaten nach dem Stand der Technik in den ersten zwei Stunden nach der Einnahme eine sehr starke Anflutung erfolgt, die dann im Laufe des Tages relativ bald wieder abklingt, so daß nach 24 Stunden die peripheren Serumspiegel nur noch etwa ein Drittel des Höchstspiegels ausmachen.

Auch die langfristige Untersuchung (6 Monate) der peripheren Serumspiegel an Östradiol hat ergeben, daß durch die erfindungsgemäße orale Zubereitungsform bei postmenopausalen Frauen die Werte von durchschnittlich 13 pg/ml auf durchschnittlich 170 pg/ml (gemessen in der Follikel phase) ansteigen. Es kommt somit zu einer guten, zuverlässigen und gleichmäßigen Substitution des bei postmenopausalen Frauen abgesunkenen Spiegels an Östradiol. Erste Untersuchungen bei manifester Osteoporose haben ergeben, daß insbesondere bei zusätzlicher Gabe von Fluorid, Vitamin D und Calcium bereits nach 6 Monaten eine Zunahme des Knochenmineralgehaltes zu beobachten ist. Diese Zunahme ist deutlich höher als bei vergleichbarer Therapie ausschließlich mit Fluorid, Vitamin D und Calcium. Die erfindungsgemäße orale Zubereitungsform ist somit nicht nur zur postmenopausalen Substitutionstherapie, sondern zusammen mit einer Basistherapie bestehend aus Fluorid, Vitamin D und Calcium auch zur Therapie der manifesten Osteoporose geeignet.

Die Herstellung der erfindungsgemäßen Weichgelatinekapseln enthaltend Östradiolvalerat, gelöst in einem physiologisch verträglichen lipophilen Medium, insbesondere in einem Pflanzenöl, vorzugsweise Soja- und/oder Sesamöl, erfolgt in an sich bekannter Weise wie ähnliche Weichgelatinekapseln. Als lipophile Medien kommen prinzipiell alle für die Herstellung von Weichgelatinekapseln üblichen Pflanzenöle, Fischöle und synthetisch hergestellten niedrigviskosen Triglyceride mittelkettiger Fettsäuren, wie Miglyol^{R} (Hesteller Dynamit Nobel), in Frage, da das Östradiolvalerat hierin gut löslich ist. Erfindungsgemäß kommt es aber offensichtlich nicht nur darauf an, daß das Östradiolvalerat in der Kapselfüllung gelöst ist. Die bisherigen Ergebnisse deuten darauf hin, daß das lipophile Medium Einfluß nimmt auf die Resorption, Aufspaltung des Esters und Anflutung des Östradiols in der Peripherie.

Das als Lösungsmittel verwendete lipophile Medium sollte daher vorzugsweise in überschüssigen Mengen eingesetzt werden. Selbst wenn konzentriertere Lösungen des Wirkstoffes in dem Medium möglich sind, sollte daher die Kapselfüllung den Wirkstoff in weniger konzentrierter Form enthalten. Konzentrationen im Bereich zwischen 0,2 und 1,2% Östradiolvalerat in einem Pflanzenöl werden daher besonders bevorzugt.

Eine typische Ausführungsform der erfindungsgemäßen oralen Zubereitungsform enthält 1 mg Östradiolvalerat in 159 mg Sesamöl, so daß die einzelne Weichgelatinekapsel ein Füllgewicht von ca. 160 mg aufweist. Selbstverständlich ist es möglich, auch entsprechende Weichgelatinekapseln mit einem Wirkstoffgehalt von 0,5 bzw. 1,5 mg herzustellen, um mit diesen Zubereitungsformen gewünschtenfalls niedrigere oder höhere Mengen applizieren zu können.

Vorzugsweise wird das erfindungsgemäße Präparat sequentiell verabreicht, wobei in den ersten 12 Tagen 1 mg Östradiolvalerat und dann 14 Tage eine Kombination aus 1,25 mg Östradiolvalerat und 5 mg eines Gestagens wie Medroxy-Progesteronacetat (MPA) genommen wird. Auch das Gestagen sollte dabei in dem lipophilen Medium gelöst vorliegen.

Beide Zubereitungsformen werden vorzugsweise mit einem magensaftresistenten Überzug versehen, so daß Freisetzung und Anflutung möglichst gleichmäßig erst im Dünndarm erfolgen.

Vergleichende pharmakodynamische Untersuchungen haben gezeigt, daß die beiden Komponenten der Kombination sich nicht negativ beeinflussen.

## Patentansprüche

1. Orale Zubereitungsform des Östradiolvalerats zur postmenopausalen Substitutionstherapie sowie der Prophylaxe und Therapie der Osteoporose, bestehend aus Weichgelatinekapseln enthaltend 0,5 bis 1,5 mg Östradiolvalerat gelöst in einem physiologisch verträglichen lipophilen Medium, insbesondere einem Pflanzenöl, vorzugsweise Soja- und/oder Sesamöl.

2. Orale Zubereitungsform gemäß Anspruch 1, dadurch gekennzeichnet, daß sie zusätzlich ca. 5 mg Gestagen, vorzugsweise Medroxy-Progesteronacetat enthält.

3. Orale Zubereitungsform gemäß Anspruch 1 oder 2, dadurch gekennzeichnet, daß sie magensaftresistent überzogen ist.

## Claims

1. An oral formulation of oestradiol valerate for the postmenopausal substitution therapy as well as prophylaxis and therapy of osteoporosis, consisting of soft gelatin capsules containing from 0.5 to 1.5 mg of oestradiol valerate dissolved in a physiologically acceptable lipophilic medium, especially a vegetable oil, and preferably soybean oil and/or sesame oil.

2. The oral formulation according to claim 1, characterized in that it additionally contains about 5 mg of gestagen, preferably medroxyprogesterone acetate.

3. The oral formulation according to claim 1 or 2, characterized in that it has been coated to be resistant to gastric juice.

## Revendications

1. Préparation orale de valérate d'estradiol pour la thérapie de substitution postménopausale, ainsi que pour la prophylaxie et la thérapie de l'ostéoporose, composée de capsules de gélatine molle contenant de 0,5 à 1,5 mg de valérate d'estradiol dissous dans un milieu lipophile physiologiquement acceptable, en particulier une huile végétale, de préférence l'huile de soja ou l'huile de sésame.

2. Préparation orale selon la revendication 1, caractérisée en ce qu'elle contient, en outre, environ 5 mg de substance gestagène, de préférence l'acétate de médroxyprogestérone.

3. Préparation orale selon la revendication 1 ou 2, caractérisée en ce qu'elle est enrobée de manière résistante au suc gastrique.
